# EUROPÄISCHE PATENTSCHRIFT

(11) **EP 1 603 606 B1**
(45) Veröffentlichungstag und Bekanntmachung des Hinweises auf die Patenterteilung: **12.05.2010**
(21) Anmeldenummer: 04719998.9
(22) Anmeldetag: 12.03.2004
(51) Int. Cl.: A61L 31/16, A61K 31/365, A61P 9/10

(54) **ENDOVASKULÄRES IMPLANTAT MIT EINER ZUMINDEST ABSCHNITTSWEISEN AKTIVEN BESCHICHTUNG AUS RATJADON UND/ODER EINEM RATJADON-DERIVAT**
ENDOVASCULAR IMPLANT WITH AN AT LEAST SECTIONAL ACTIVE COATING MADE OF RADJADONE AND/OR A RATJADONE DERIVATIVE
IMPLANT ENDOVASCULAIRE PRESENTANT UN REVETEMENT AU MOINS PARTIELLEMENT ACTIF EN RATJADON ET/OU EN UN DERIVE DE RATJADON

(30) Priorität: 18.03.2003 DE 10311729
(43) Veröffentlichungstag der Anmeldung: 14.12.2005
(73) Patentinhaber: Schultheiss, Heinz-Peter, 14163 Berlin (DE)
(72) Erfinder: Schultheiss, Heinz-Peter, 14163 Berlin (DE)
(74) Vertreter: Lindner-Vogt, Karin L.
(86) Internationale Anmeldenummer: PCT/EP2004/002621
(87) Internationale Veröffentlichungsnummer: WO 2004/082454

(56) Entgegenhaltungen:
- WO-A-02/064587
- WO-A-03/099348
- WITZENBICHLER B ET AL: "Ratjadone is a potent inhibitor of vascular smooth muscle cell proliferation." EUROPEAN HEART JOURNAL, Bd. 24, Nr. Abstract Supplement, 30. September 2003 (2003-09-30), Seite 333, XP009034690 & CONGRESS OF THE EUROPEAN SOCIETY OF CARDIOLOGY; VIENNA, AUSTRIA; AUGUST 30-SEPTEMBER 03, 2003 ISSN: 0195-668X
- WITZENBICHLER BERNHARD J ET AL: "Ratjadone is a potent inhibitor of coronary smooth muscle cell proliferation and induces cell differentiation." CIRCULATION, Bd. 108, Nr. 17 Supplement, 28. Oktober 2003 (2003-10-28), Seiten IV-194, XP009034689 & AMERICAN HEART ASSOCIATION SCIENTIFIC SESSIONS 2003; ORLANDO, FL, USA; NOVEMBER 09-12, 2003 ISSN: 0009-7322

## Beschreibung

Die Erfindung betrifft ein endovaskuläres Implantat mit einer zumindest abschnittsweisen aktiven Beschichtung aus Ratjadon und/oder einem Ratjadon-Derivat, die Verwendung der Substanzen zur Herstellung eines Arzneimittels zur Hemmung der Restenose und eine Formulierung mit den genannten Substanzen.

Zum Hintergrund der Erfindung ist darauf hinzuweisen, dass eine der häufigsten Todesursachen in Westeuropa und Nordamerika koronare Herzkrankheiten sind. Nach neuen Erkenntnissen sind vor allem entzündliche Prozesse treibende Kraft der Arteriosklerose. Initiiert wird der Prozess vermutlich durch die vermehrte Einlagerung von Lipoproteinen geringer Dichte in die Intima der Gefäßwand. Nach dem Eindringen in die Intima werden die LDL-Partikel durch Oxidantien chemisch modifiziert. Die modifizierten LDL-Partikel veranlassen wiederum die Endothelzellen, die die inneren Gefäßwände auskleiden, das Immunsystem zu aktivieren. In der Folge treten Monocyten in die Intima ein und reifen zu Makrophagen heran. Im Zusammenspiel mit den ebenfalls eintretenden T-Zellen werden Entzündungsmediatoren, wie Immunbotenstoffe und proliferativ wirkende Substanzen freigesetzt, und die Makrophagen beginnen die modifizierten LDL-Partikel aufzunehmen. Die sich bildenden Lipidläsionen aus T-Zellen und den mit LDL-Partilceln gefüllten Makrophagen, die aufgrund ihres Aussehens Schaumzellen genannt werden, stellen eine Frühform der arteriosklerotischen Plaque dar. Die Entzündungsreaktion in der Intima veranlasst durch entsprechende Entzündungsmediatoren glatte Muskelzellen der weiter außen liegenden Media der Gefäßwand bis unter die Endothelzellen zu wandern. Dort vermehren sie sich und bilden eine fibröse Deckschicht aus dem Faserprotein Kollagen, die den darunter liegenden Lipidkern aus Schaumzellen gegen die Blutbahn abgrenzt. Die dann vorliegenden tiefgreifenden strukturellen Veränderungen in der Gefäßwand werden zusammenfassend als Plaque bezeichnet.

Der arteriosklerotische Plaque expandiert zunächst relativ wenig in Richtung der Blutbahn, da diese sich kompensatorisch erweitern kann. Mit der Zeit kommt es jedoch zu einer Verengung des Blutkanals (Stenose), deren erste Anzeichen bei körperlicher Belastung auftreten. Die verengte Arterie kann sich dann nicht mehr ausreichend weiten, um das zu versorgenden Gewebe besser zu durchbluten. Ist eine Herzarterie betroffen, so klagt der Patient häufig über ein Druck- und Engegefühl hinter dem Brustbein (Angina pectoris). Bei anderen Arterien sind schmerzhafte Kämpfe ein häufig auftretendes Indiz für die Stenose.

Die Stenose kann letztendlich zu einem völligen Verschließen der Blutbahn führen (Herzinfarkt, Schlaganfall). Allein durch die Plaquebildung tritt dies nach neueren Untersuchungen in etwa 15 Prozent der Fälle auf. Darüber hinaus scheint der durch bestimmte Entzündungsmediatoren aus den Schaumzellen veranlasste allmähliche Abbau der fibrösen Deckschicht aus Kollagen ein entscheidender Zusatzfaktor zu sein. Reißt die fibröse Deckschicht auf, so kann der Lipidkern unmittelbar mit dem Blut in Kontakt kommen. Da in Folge der Entzündungsreaktion gleichzeitig Gewebsfaktoren (TF tissue factor) in den Schaumzellen produziert werden, die sehr potente Auslöser der Gerinnungskaskade sind, kann der sich bildende Blutpfropf das Blutgefäß verschließen.

Seit mehr als zwanzig Jahren sind nicht-operative Methoden zur Stenose-Behandlung etabliert, bei denen u.a. durch Ballondilatation (PTCA Perkutane Transluminale Coronare Angioplastie) das Blutgefäß wieder aufgeweitet wird. Mit dem Aufweiten des Blutgefäßes entstehen allerdings Verletzungen in der Gefäßwand, die zwar problemlos verheilen, jedoch in bis zu 60% der Fälle durch das ausgelöste Zellwachstum zu Wucherungen führen (Proliferation), die letztendlich zu einem erneuten Gefäßverschluss (Restenose) führen. Die Aufweitung beseitigt auch nicht die physiologischen Ursachen der Stenose, also die Veränderungen in der Gefäßwand. Eine weitere Ursache der Restenose ist die Elastizität des gedehnten Blutgefäßes. Nach dem Entfernen des Ballons zieht sich das Blutgefäß übermäßig zusammen, so dass der Gefäßquerschnitt verringert wird (Obstruktion). Letzterer Effekt kann nur durch Platzierung eines Stents vermieden werden. Durch den Einsatz von Stents kann zwar ein optimaler Gefäßquerschnitt erreicht werden, allerdings führt der Einsatz von Stents ebenfalls zu kleinsten Verletzungen, die die Proliferation induzieren und damit letztendlich die Restenose auslösen können.

Mittlerweile bestehen umfangreiche Erkenntnisse zum zellbiologischen Mechanismus und zu den auslösenden Faktoren der Stenose und Restenose. Die Restenose entsteht - wie bereits erläutert - als Reaktion der Gefäßwand auf die Dehnung des artierosklerotischen Plaque. Über komplexe Wirkmechanismen wird die lumengerichtete Migration und Proliferation der glatten Muskelzellen der Media und der Adventitia induziert (neointimale Hyperplasie). Unter Einfluss verschiedener Wachstumsfaktoren produzieren die glatten Muskelzellen eine Deckschicht aus Matrixproteinen (Elastin, Kollagen, Proteoglykane), deren ungesteuertes Wachstum allmählich zu einer Einengung des Lumens führen kann. Systematisch medikamentöse Therapieeinsätze sehen u.a. die orale Verabreichung von Calzium-Antagonisten, ACE-Hemmern, Antikoagulantien, Antiaggregantien, Fischölen, antiproliferativen Substanzen, antiinflammatorischen Substanzen und Serotonin-Antagonisten vor, signifikante Reduktionen der Restenoseraten wurden auf diesem Wege bisher jedoch nicht erreicht.

Das sogenannte Konzept des Local Drug Delivery (LDD) sieht nun vor, den oder die Wirkstoffe unmittelbar am Ort des Geschehens und begrenzt auf dieses Arial freizusetzen. Dazu wird im allgemeinen eine dem Blutgefäß zugewandte Oberfläche des endovaskulären Implantates, also insbesondere eines Stents, mit einer aktive Beschichtung versehen. Die aktive Komponente der Beschichtung in Form eines therapeutischen Wirkstoffes kann direkt an der Oberfläche des Implantates gebunden oder in einem geeigneten Arzneistoffträger eingebettet sein. Im letzteren Fall wird durch Diffusion und gegebenenfalls allmählichen Abbau des biodegradierbaren Trägers der Wirkstoff freigesetzt.

Als Wirkstoffe und Wirkstofkombinationen wurden zahlreiche Präparate vorgeschlagen, jedoch ist der bisher in therapeutischen Versuchen nachgewiesene Effekt nur mäßig.

Der Erfindung liegt die Aufgabe zugrunde, sowohl die Behandlung von Stenosen als auch die Prävention von Restenosen weiter zu verbessern und ein in diesem Zusammenhang besonders geeignetes endovaskuläres Implantat mit einer aktiven Beschichtung bereitzustellen.

Diese Aufgabe wird durch ein endovaskuläres Implantat einer zumindest abschnittsweisen aktiven Beschichtung gelöst, wobei die aktive Beschichtung eine (re-)stenosehemmende Substanz folgender Formel beinhaltet: 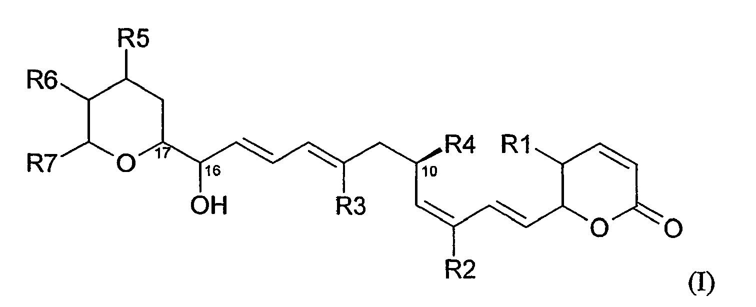
wobei R1, R2 und R3 unabhängig voneinander ausgewählt sind aus der Gruppe H, CH₃ und C₂H₅,
R4 CH₃ oder C₂H₅ ist,
R5 H oder OH ist, und
R6 und R7 unabhängig voneinander ausgewählt sind aus der Gruppe H, CH₃, C₂H₅, n-C₃H₇, iso-C₃H₇, Vinyl, CHCHCH₃ und C(CH₃)CH₂.

Die aktive Beschichtung beinhaltet als (re-)stenosehemmende Substanz mithin den Wirkstoff Ratjadon, das seit geraumer Zeit grundsätzlich als antibiotische, antitumorale oder zellwachstumshemmende Verbindung bekannt ist. Es wird in diesem Zusammenhang auf die DE 196 36 721 A1 und DE 101 06 647 A1 verwiesen. Insbesondere die letztgenannte Druckschrift gibt auch das allgemeine Syntheseschema für die Ratjadon-Derivate an.

Überraschenderweise wurde nun entdeckt, dass der Naturstoff Ratjadon und Ratjadon-Derivate das Wachstum aortaler, glatter Gefäßmuskelzellen des Menschen hemmen. Dieser Effekt tritt schon ab 1 bis 100 nM, bevorzugt 5 bis 50 nM, auf, so dass geringste lokale Konzentrationen zur wirkungsvollen Hemmung der Restenose im Bereich eines Implantates bereits ausreichend sind. Damit werden weiterreichende Nebenwirkungen nahezu ausgeschlossen.

Bevorzugtermaßen handelt es sich bei einem als Wirkstoff verwendeten Ratjadon-Derivat um das (+)-Ratjadon. Der Naturstoff hat sich in ersten Versuchen als besonders potent, dass heißt schon in geringsten Wirkstoffkonzentrationen als pharmakologisch aktiv erwiesen. Bevorzugte Varianten sehen ferner vor, dass das C10- und C17-Kohlenstoffatom R-konfiguriert sind, wenn das C16-Kohlenstoffatom R-konfiguriert ist und gleichzeitig weder R5 noch R6 noch R7 in der oben bezeichneten Formel I H sind. Andernfalls können diese Reste R5, R6 und R7 auch H sein. Die genannten Derivate zeichnen sich durch ein potentiell verbesserte Verträglichkeit im Vergleich zum Naturstoff aus.

Nach einer bevorzugten Variante der Erfindung werden Ratjadon und seine Derivate in einen Arzneistoffträger eingebettet. Hierdurch lässt sich die Herstellung der beschichteten Implantate vereinfachen und die Freisetzung des Arzneistoffes steuern. Zudem kann ein unerwünschtes Abblättern des Wirkstoffes während der Implantation, insbesondere der Dilatation des Stents, wirkungsvoll unterdrückt werden. Es versteht sich von selbst, dass der Arzneistoffträger biokompatibel sein muss. Bevorzugt ist der Arzneistoffträger zusätzlich auch biodegradierbar, so dass über sein Abbauverhalten eine gezielte Dosierung des Arzneistoffes möglich ist. In diesem Zusammenhang hat sich die Verwendung von Glykosaminoglykanen, insbesondere Hyaluronsäure, oder von Derivaten dieser Substanzen als besonders günstig erwiesen.

Glykosaminoglykane sind negativ geladene Polysaccharide, die aus 1,4-verknüpften Disaccharid-Einheiten bestehen. Ein Baustein dieser Einheit ist eine Uronsäure (z.B. D-Glucoronsäure, L-Iduronsäure), die über eine β-(1→3)-Bindung mit einem Aminozucker verbunden ist.

Eine Schichtdicke der aktiven Beschichtung liegt im Falle von Arzneistoffträgem mit eingebettetem Wirkstoff vorzugsweise zwischen 3 und 30 µm, insbesondere zwischen 8 und 15 µm. Eine Gewichtsmasse pro Implantat, also das Gewicht des Arzneistoffträgers plus Wirkstoff, liegt vorzugsweise im Bereich von 0,3 bis 2 mg, insbesondere 0,5 bis 1 mg. Mit den gewählten Bereichen lässt sich eine hohe lokale Wirkung erzielen, ohne dass es zu den gefürchteten Nebenwirkungen in Niere, Galle usw. kommen kann. Derartig dünne Beschichtungen neigen auch nicht zur Rissbildung und wirken demnach einem Abblättern bei mechanischer Beanspruchung entgegen.

Wird ein biodegradierbarer Arzneistoffträger eingesetzt, so kann die Elutionscharall-teristik insbesondere durch eine Variation des Vernetzungsgrades der Polymermatrix oder eine Variation des Polymerisationsgrades beeinflusst werden. Neben der Degradation des Trägers sind Diffusionsvorgänge für die Elution des Wirkstoffes maßgebend. Strukturelle Eigenschaften des Trägers und des Wirkstoffs beeinflussen neben vielen weiteren Faktoren die Diffusionsgeschwindigkeit.

Bevorzugtermaßen kann zwischen der aktiven Beschichtung und einem Grundkörper des Implantats eine passive Beschichtung vorgesehen sein, die amorphes Siliciumcarbid enthält. Damit kann das Haftungsvermögen der aktiven Beschichtung an der Oberfläche des Implantats verbessert werden. Zudem mindert die passive Beschichtung für sich allein bereits die neointimale Proliferation.

Ferner ist es vorteilhaft, wenn ein Grundkörper des Implantates aus zumindest einem Metall oder zumindest einer Metalllegierung geformt ist. Vorteilhaft ist weiterhin, wenn das Metall oder die Metalllegierung zumindest teilweise biodegradierbar ist. Die biodegradierbare Metalllegierung kann insbesondere eine Magnesiumlegierung sein. Der Stent wird nach der biodegradierbaren Variante mit der Zeit vollständig abgebaut und damit verschwinden auch mögliche Auslöser für eine inflammatorische und proliferative Reaktion des umgebenden Gewebes.

Die Erfindung betrifft ferner eine Formulierung zur (Re-)stenose-Hemmung, die eine zur Hemmung der (Re-)stenose ausreichende Konzentration einer Ratjadon-Substanz nach einem oder mehreren der Ansprüche 1 bis 4 und einen pharmazeutisch akzeptablen Träger aufweist.

Weitere Merkmale, Einzelheiten und Vorteile der Erfindung ergeben sich aus der nachfolgenden Beschreibung, in der ein Ausführungsbeispiel anhand der beigefügten Zeichnungen näher erläutert wird. Es zeigen:
- Fig. 1: eine Draufsicht auf ein endovaskuläres Implantat in Form eines Stents in abgewickelter Darstellung und
- Fig. 2: einen vergrößerten Detailschnitt durch das Implantat gemäß Schnitt- linie II-II nach Fig. 1.

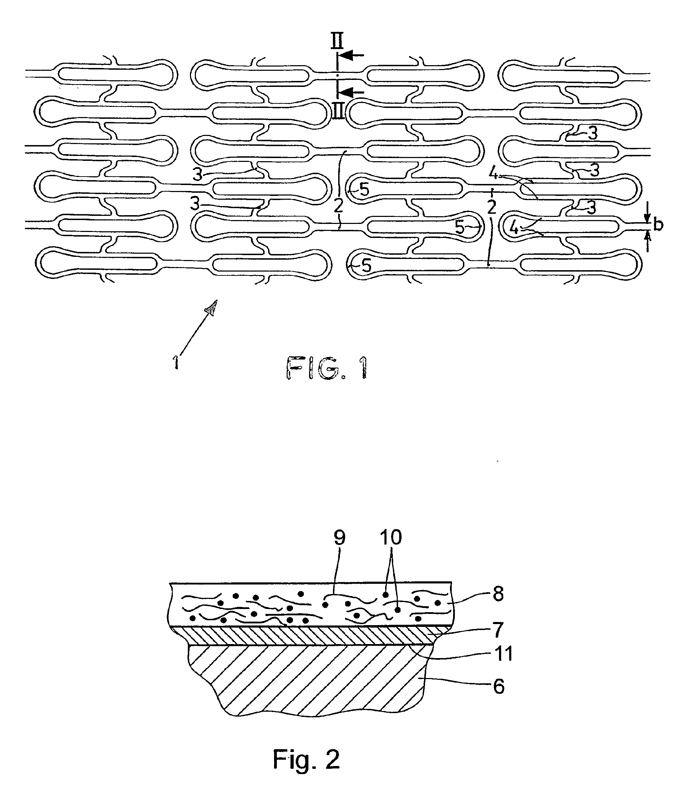

Wie aus Fig. 1 deutlich wird, besteht ein dilatierbarer Stent 1 aus einem fein strukturierten Netz von Längsstegen 2 und diese verbindenden Querstegen 3. Die Längsstege 2 verzweigen sich dabei in einander parallele Stränge 4, die jeweils am Ende über einen Bogen 5 paarweise miteinander verbunden sind. Links und rechts bezogen auf Fig. 1 setzten sich die Längsstege 2 mit Ihren Verzweigungssträngen 4 bis zum Ende des insgesamt rohrförmigen Stents 1 fort. In Richtung der Querstege 3 ist die Struktur zylindrisch gebogen, sodass die oben bezüglich Fig. 1 auslaufenden Querstege 3 in die unten auslaufenden Querstege 3 übergehen. In Ihrer Größenordnung liegen die Breiten b der Stege 2, 3 im Submillimeterbereich.

Aus Fig. 2 ist der Schichtaufbau der Struktur des Stents 1 erkennbar. Als tragendes Teil dient dabei ein Grundkörper 6, der aus Metall oder einer Metalllegierung geformt sein kann.

Soll der gesamte Stent 1 biodegradierbar sein, so kann der Grundkörper 6 insbesondere auf Basis eines biodegradierbaren Metalls oder einer biodegradierbaren Metalllegierung hergestellt werden. Besonders geeignet ist eine biodegradierbare Magnesiumlegierung. Derartige Materialien sind bereits ausreichend im Stand der Technik beschrieben worden, so dass auf eine gesonderte Darstellung verzichtet wird. Es wird in diesem Zusammenhang insbesondere auf die Offenbarung der DE 198 56 983 A1 der Anmelderin verwiesen.

Auf diesem Grundkörper 6 sind eine noch näher zu erläuternde passive Beschichtung 7 und eine wiederum darauf sitzende aktive Beschichtung 8 aufgebracht, die aus einem Arzneistoffträger 9 und einer darin eingebetteten, Restenose-hemmenden Substanz 10 besteht. Letztere ist in Fig. 2 durch Punkte versinnbildlicht.

Die passive Beschichtung 7 sorgt für eine besonders hohe Haftung der aktiven Beschichtung 8 auf der Oberfläche 11 des Stent-Grundkörpers 6. Die passive Beschichtung 7 ist aus amorphem Siliziumcarbid aufgebaut. Die Herstellung derartiger Strukturen ist aus dem Stand der Technik, insbesondere aus der Patentschrift DE 44 29 380 C1 der Anmelderin bekannt. Auf den Offenbarungsgehalt dieser Druckschrift wird voll umfänglich verwiesen, sodass sich nähere Ausführungen zur Herstellung der passiven Beschichtung 7 an dieser Stelle erübrigen.

Der erwähnte Arzneistoffträger 9 in der aktiven Beschichtung 8 wird durch Hyaluronsäure gebildet, die biokompatibel ist und eine kontrollierte Freisetzung der darin eingebetteten Wirkstoffsubstanz 10 erlaubt. Darüber hinaus dient der Arzneistoffträger 9 dazu, ein Abblättern der aktiven Beschichtung 8 bei der Dilatation oder Einbringung des Stents 1 in ein arerielles Gefäß zu verhindern. Das Stentdesign sollte dabei derart angepasst sein, dass ein möglichst großflächiger Kontakt zur Gefäßwand besteht. Dies begünstigt eine gleichmäßige Elution des Wirkstoff, die Untersuchungen zufolge im wesentlichen diffusionskontrolliert ist. Bereiche hoher mechanischer Verformbarkeit sind vorzugsweise bei der Beschichtung 7, 8 auszusparen, da hier die Gefahr des Abblättems der Beschichtung 7, 8 erhöht ist. Alternativ oder in Ergänzung hierzu kann das Stentdesign derart vorgegeben werden, dass bei mechanischer Belastung, d.h. in der Regel bei der Dilatation des Stents, eine möglichst gleichmäßige Verteilung der auftretenden Kräfte über die gesamte Stentoberfläche gegeben ist. Auf diese Weise können lokale Überbeanspruchungen und damit eine Rissbildung oder gar Abblättern der Beschichtung vermieden werden.

Die eigentliche aktive Substanz 10 im Arzneistoffträger 9 ist im konkreten Ausführungsbeispiel durch ein Ratjadon-Derivat der folgenden Formulierung gebildet: 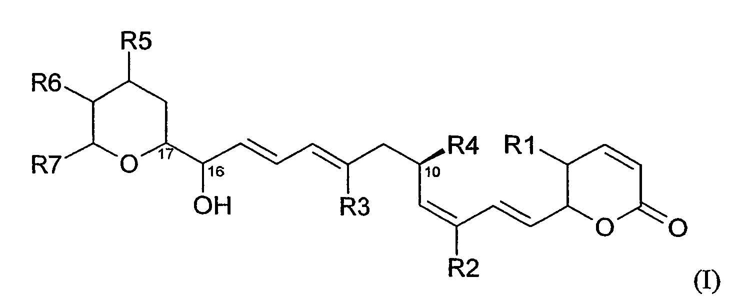
wobei R1, R2 und R3 unabhängig voneinander ausgewählt sind aus der Gruppe H, CH₃ und C₂H₅,
R4 CH₃ oder C₂H₅ ist,
R5 H oder OH ist, und
R6 und R7 unabhängig voneinander ausgewählt sind aus der Gruppe H, CH₃, C₂H₅, n-C₃H₇, iso-C₃H₇, Vinyl, CHCHCH₃ und C(CH₃)CH₂.

Dabei sind im konkreten Beispiel das C10-, C17- und C16-Kohlenstoffatom R-konfiguriert und die Reste R5, R6 und R7 nicht durch Wasserstoff H besetzt.

Wenn der Arzneistoffträger 9 biodegradierbar ist, kann die Elutionscharakteristik des Wirkstoffes durch Variation des Vernetzungsgrades der Polymermatrix oder eine Variation des Polymerisationsgrades beeinflusst werden. Das Vorgehen bietet sich insbesondere für den genannten Arzneistoffträger Hyaluronsäure oder Polylactid an. Mit steigendem Vernetzungsgrad und steigender Molekularmasse des Polymers verlängert sich im allgemeinen auch der Zeitraum, in dem der Wirkstoff freigesetzt wird. Die Elutionscharakteristik einer derartigen aktiven Beschichtung wird vorzugsweise derart eingestellt, dass 10 bis 30 %, insbesondere 15 bis 25 %, des Wirkstoffes innerhalb der ersten zwei Tage freigesetzt wird. Der Rest des verbleibenden Wirkstoffes soll - ebenfalls gesteuert über Diffusions- und Degradationsvorgänge - sukzessive innerhalb der ersten Monate abgegeben werden. Es hat sich überraschender Weise gezeigt, dass diese an sich relativ kurzen Zeiträume bereits eine wirkungsvolle Unterdrückung der neointimalen Proliferation ermöglichen.

Die aktive Beschichtung 8 kann ferner im Aufbau strukturiert sein. Beispielsweise kann in den äußeren Bereichen der aktiven Beschichtung 8 ein geringerer Vernetzungsgrad als in den weiter innen liegenden Bereichen vorgesehen sein. Hierdurch kann der Abbau der aktiven Beschichtung 8 nach Implantation zunächst schneller erfolgen und bei gleichmäßiger Wirkstofflconzentration in der aktiven Beschichtung 8 eine insgesamt höhere Anfangsdosis als in dem verbleibenden Zeitraum freigesetzt werden. Alternativ oder ergänzend kann dieser Effekt durch Vorgabe lokal unterschiedlicher Konzentrationen der Wirkstoffsubstanz 10 in der aktiven Beschichtung 8 erreicht werden, indem beispielsweise die obersten Bereiche der Beschichtung 8 höhere Wirkstoffkonzentrationen aufweisen.

Die Herstellung der aktiven Beschichtung 8 wird mit Hilfe eines Rotationszerstäubers durchgerührt, der einen Nebel aus mikrofeinen Partikeln erzeugt. Alternativ können auch Ultraschallzerstäuber eingesetzt werden. Die Beschichtung erfolgt schrittweise in zahlreichen Zyklen, die aus einem Benetzungsschritt des Stents im erzeugten Sprühnebel und einem anschließenden Trocknungsschritt des Niederschlags auf dem Stent durch Abblasen bestehen. Durch das mehrstufige Herstellungsverfahren lassen sich beliebige Schichtdicken und - sofern gewünscht - Konzentrationsgradienten des oder der Wirkstoffe in einzelnen Lagen der aktiven Beschichtung 8 erzeugen. Sofern gewünscht, können auf diese Weise auch mehrschichtige Systeme - beispielsweise für die Kombination von Ratjadon und Ratjadon-Derivaten - erzeugt werden, die aufeinanderfolgend aufgebracht werden.

Eine Sterilisation des Stents erfolgt durch Elektronenbeschuss, wobei ein teilweises Cracken der Polymerketten eines gegebenenfalls vorhandenen polymeren Träges bei hohen Molekulargewichten des Polymers in Kauf genommen werden kann. Die kinetische Energie der Elektronen liegt etwa im Bereich von 4 bis 5 MeV, da bei diesen Werten noch eine ausreichende Sterilisation bei nur geringer Eindringtiefe sichergestellt ist. Die Dosierung bewegt sich im Bereich von 15 bis 35 kGy pro Stent. Untersuchungen zeigten, dass keine oder nur eine minimale Minderung der biologischen Aktivität der Wirkstoffe durch das Sterilisationsverfahren erfolgt.

Die erzeugten Schichtdicken der aktiven Beschichtung 8 liegen in der Regel im Bereich von 5 bis 30 µm. Besonders günstig sind Schichtdicken im Bereich von 8 bis 15 µm, da hier eine weitestgehende Abdeckung der Oberfläche 11 des Stents 1 gewährleistet ist und noch nicht mit strukturellen Problemen wie Rissbildung und dergleichen gerechnet werden muss. Insgesamt werden pro endovaskulärem Implantat etwa 0,3 bis 2 mg, insbesondere 0,5 bis 1 mg an Beschichtungsmaterial aufgebracht, wenn die aktive Beschichtung 8 einen Arzneistoffträger beinhaltet.

Zur Hemmung der Restenose beinhaltet die Beschichtung eine ausreichende Konzentration an Ratjadon und/oder eines Ratjadon-Derivats. Die Elutionscharakteristik wird in vorgenannter Weise derart vorgegeben, dass die Konzentration der Substanz(en) in unmittelbarer Nachbarschaft der Beschichtung etwa 1 bis 100 nM, insbesondere 5 bis 50 nM beträgt. Diese niedrigen Konzentrationsbereiche haben in Versuchen bereits einen restenosehemmenden Effekt gezeigt.

## Patentansprüche

1. Endovaskuläres Implantat mit einer zumindest abschnittsweisen aktiven Beschichtung, **dadurch gekennzeichnet, dass** die aktive Beschichtung eine (re-)-stenose-hemmende Substanz folgender Formel beinhaltet: 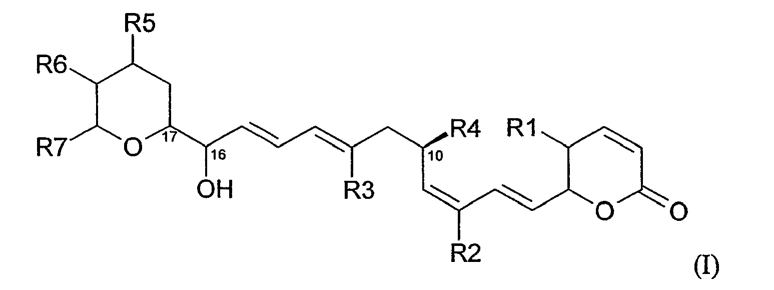
wobei R1, R2 und R3 unabhängig voneinander ausgewählt sind aus der Gruppe H, CH₃ und C₂H₅,
R4 CH₃ oder C₂H₅ ist,
R5 H oder OH ist, und
R6 und R7 unabhängig voneinander ausgewählt sind aus der Gruppe H, CH₃, C₂H₅, n-C₃H₇, iso-C₃H₇, Vinyl, CHCHCH₃ und C(CH₃)CH₂.

2. Implantat nach Anspruch 1, **dadurch gekennzeichnet, dass** die Substanz (+)-Ratjadon ist.

3. Implantat nach Anspruch 1, **dadurch gekennzeichnet, dass** C10 und C 17 R-konfiguriert sind, wenn C16 R-konfiguriert ist und gleichzeitig weder R5, noch R6, noch R7 H sind.

4. Implantat nach Anspruch 1, **dadurch gekennzeichnet, dass** R5, R6 und R7 H sind.

5. Implantat nach einem der vorhergehenden Ansprüche, **dadurch gekennzeichnet, dass** die aktive Beschichtung (8) einen Arzneistoffträger (9), in dem der Wirkstoff (10) eingebettet ist, umfasst.

6. Implantat nach Anspruch 5, **dadurch gekennzeichnet, dass** der Arzneistoffträger (9) ein Glykosaminoglykan oder derivatisiertes Glykosaminoglykan ist.

7. Implantat nach Anspruch 6, **dadurch gekennzeichnet, dass** das Glykosaminoglykan Hyaluronsäure oder eine derivatisierte Hyaluronsäure ist.

8. Implantat nach einem der vorhergehenden Ansprüche, **dadurch gekennzeichnet, dass** eine Schichtdicke der aktiven Beschichtung (8) 3 bis 30 µm beträgt.

9. Implantat nach Anspruch 8, **dadurch gekennzeichnet, dass** die Schichtdicke der aktiven Beschichtung (8) 8 bis 15 µm beträgt.

10. Implantat nach mindestens einem der Ansprüche 5, 6 oder 7, **dadurch gekennzeichnet, dass** eine Gesamtmasse der aktiven Beschichtung (8) aus Arzneistoffträger (9) und Wirkstoff (10) 0,3 bis 2 mg beträgt.

11. Implantat nach Anspruch 10, **dadurch gekennzeichnet, dass** die Gesamtmasse der aktiven Beschichtung (8) aus Arzneistoffträger (9) und Wirkstoff (10) 0,5 bis 1 mg beträgt.

12. Implantat nach einem oder mehreren der Ansprüche 5 bis 11, **dadurch gekennzeichnet, dass** der Arzneistoffträger (9) biodegradierbar ist.

13. Implantat nach einem der vorhergehenden Ansprüche, **dadurch gekennzeichnet, dass** zwischen der aktiven Beschichtung (8) und einem Grundkörper (6) des Implantates eine passive Beschichtung (7), die amorphes Siliziumkarbid enthält, vorgesehen ist.

14. Implantat nach einem der vorhergehenden Ansprüche, **dadurch gekennzeichnet, dass** der Grundkörper (6) des Implantates (1) aus zumindest einem Metall oder zumindest einer Metalllegierung geformt ist.

15. Implantat nach Anspruch 14, **dadurch gekennzeichnet, dass** das Metall oder die Metalllegierung zumindest teilweise biodegradierbar ist.

16. Implantat nach Anspruch 15, **dadurch gekennzeichnet, dass** die biodegradierbare Metalllegierung eine Magnesiumlegierung ist.

17. Formulierung zur (Re-)stenose-Hemmung, umfassend
(a) eine zur Hemmung der Restenose ausreichende Konzentration einer Substanz nach einem oder mehreren der Ansprüche 1 bis 4, und
(b) einen pharmazeutisch akzeptablen Träger.

18. Verwendung einer Substanz nach einem oder mehreren der Ansprüche 1 bis 4 zur Herstellung eines Arzneimittels zur Hemmung der Restenose.

## Claims

1. An endovascular implant having an active coating present in at least some areas, **characterized in that** the active coating contains a (re-) stenosis inhibiting substance with the following formula: 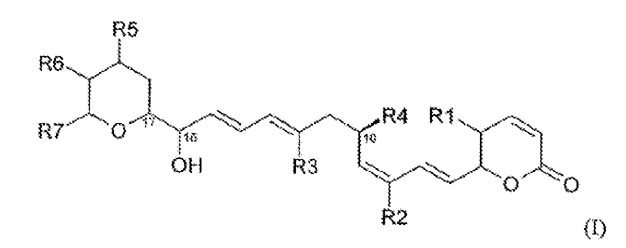
wherein R1, R2, and R3 are selected independently of each other from a group containing H, CH₃, and C₂H₅,
R4 is CH₃ or C₂H₅,
R5 is H or OH, and
R6 and R7 are selected independently of each other from a group containing H, CH₃, C₂H₅, n-C₃H₇, iso-C₃H₇, vinyl, CHCHCH₃, and C(CH₃)CH₂

2. The implant according to claim 1, **characterized in that** the substance is (+)-ratjadone.

3. The implant according to claim 1, **characterized in that** C10 and C17 are R-configured if C16 is R-configured, and at the same time neither R5 nor R6, nor R7 are H.

4. The implant according to claim 1, **characterized in that** R5, R6, and R7 are H.

5. The implant according to one of the previous claims, **characterized in that** the active coating (8) comprises an excipient (9) into which the active pharmaceutical ingredient (10) is incorporated.

6. The implant according to claim 5, **characterized in that** the excipient (9) is a glycosaminoglycan or a derivative thereof.

7. The implant according to claim 6, **characterized in that** the glycosaminoglycan is hyaluronic acid or a derivative of hyaluronic acid.

8. The implant according to one of the previous claims, **characterized in that** the layer thickness of the active coating (8) is 3 to 30 µm.

9. The implant according to claim 8, **characterized in that** the layer thickness of the active coating (8) is 8 to 15 µm.

10. The implant according to at least one of the claims 5, 6, or 7, **characterized in that** the total mass of the active coating (8) made of the excipient (9) and the active pharmaceutical ingredient (10) is 0.3 to 2 mg.

11. The implant according to claim 10, **characterized in that** the total mass of the active coating (8) made of the excipient (9) and the active pharmaceutical ingredient (10) is 0.5 to 1 mg.

12. The implant according to one or more of the claims 5 to 11, **characterized in that** the excipient (9) is biodegradable.

13. The implant according to one of the previous claims, **characterized in that** a passive coating (7) containing amorphous silicon carbide, is present between the active coating (8) and a base body (6) of the implant.

14. The implant according to one of the previous claims, **characterized in that** the base body (6) of the implant (1) is formed from at least one metal or at least one metal alloy.

15. The implant according to claim 14, **characterized in that** the metal or the metal alloy is at least partially biodegradable.

16. The implant according to claim 15, **characterized in that** the biodegradable metal alloy is an alloy of magnesium.

17. A formulation for the inhibition of (re-)stenosis, having
(a) a concentration of a substance according to one or more of the claims 1 to 4, the concentration being sufficient to inhibit restenosis,
and
(b) a pharmaceutically acceptable excipient.

18. Use of a substance according to one or more of the claims 1 to 4 for the production of a pharmaceutical for the inhibition of restenosis.

## Revendications

1. Implant endovasculaire avec un revêtement actif au moins par sections, **caractérisé par le fait que** le revêtement actif contient une substance empêchant une (re)sténose de la formule suivante : 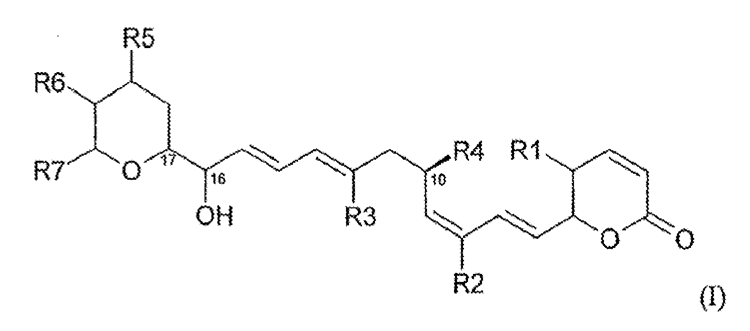
dans laquelle R1, R2 et R3 sont choisis indépendamment les uns des autres dans le groupe constitué par H, CH3 et C₂H₅,
R4 représente CH₃ ou C₂H₅,
R5 représente H ou OH, et
R6 et R7 sont choisis indépendamment l'un de l'autre dans le groupe constitué par H, CH₃, C₂H₅, n-C₃H₇, vinyle , CHCHCH₃ et C(CH₃)CH₂.

2. Implant selon la revendication 1, **caractérisé par le fait que** la substance est le (+)-Ratjadon.

3. Implant selon la revendication 1, **caractérisé par le fait que** C10 et C17 sont en configuration R lorsque C16 est en configuration R et simultanément ni R5 ni R6 ni R7 ne représentent H.

4. Implant selon la revendication 1, **caractérisé par le fait que** R5, R6 et R7 représentent H.

5. Implant selon l'une des revendications précédentes, **caractérisé par le fait que** le revêtement actif (8) comprend un support de médicament (9) dans lequel l'agent actif (10) est incorporé.

6. Implant selon la revendication 5, **caractérisé par le fait que** le support de médicament (9) est un glycosaminoglycane ou un dérivé de glycosaminoglycane.

7. Implant selon la revendication 6, **caractérisé par le fait que** le glycosaminoglycane est l'acide hyaluronique ou un dérivé de l'acide hyaluronique

8. Implant selon l'une des revendications précédentes, **caractérisé par le fait qu**'une épaisseur de couche du revêtement actif (8) s'élève à 3 à 30µm.

9. Implant selon la revendication 8, **caractérisé par le fait que** l'épaisseur de couche du revêtement actif (8) s'élève à 8 à 15µm.

10. Implant selon au moins l'une des revendications 5, 6 ou 7, **caractérisé par le fait qu**'une masse totale du revêtement actif (8) composé du support de médicament (9) et de l'agent actif (10) s'élève à 0.3 à 2 mg.

11. Implant selon la revendication 10, **caractérisé par le fait que** la masse totale du revêtement actif (8) composé du support de médicament (9) et de l'agent actif (10) s'élève à 0.5 à 1 mg.

12. Implant selon l'une ou plusieurs des revendications 5 à 11, **caractérisé par le fait que** le support de médicament (9) est biodégradable.

13. Implant selon l'une des revendications précédentes, **caractérisé par le fait qu**'entre le revêtement actif (8) et un corps de base (6) de l'implant est prévu un revêtement passif (7) qui contient du carbure de silicium amorphe.

14. Implant selon l'une des revendications précédentes, **caractérisé par le fait que** le corps de base (6) de l'implant (1) est formé d'au moins un métal ou d'au moins un alliage métallique.

15. Implant selon la revendication 14, **caractérisé par le fait que** le métal ou l'alliage métallique est au moins partiellement biodégradable.

16. Implant selon la revendication 15, **caractérisé par le fait que** l'alliage métallique biodégradable est un alliage de magnésium.

17. Formulation pour empêcher une (re)sténose comprenant
(a) une concentration suffisante pour empêcher la resténose d'une substance telle que définie à l'une ou plusieurs des revendications 1 à 4, et
(b) un support pharmaceutiquement acceptable.

18. Utilisation d'une substance telle que définie à l'une ou plusieurs des revendications 1 à 4 pour la fabrication d'un médicament pour empêcher la resténose.
